(19) Europäisches Patentamt
European Patent Office
Office européen des brevets

(11) Publication number : 0 353 898 B1

(12) # EUROPEAN PATENT SPECIFICATION

(45) Date of publication of patent specification : **30.12.92 Bulletin 92/53**

(21) Application number : **89307222.3**

(22) Date of filing : **17.07.89**

(51) Int. Cl.$^5$ : **C07C 69/017,** C07C 67/29, C07C 67/297, C07C 49/825, C07C 45/46, C07C 67/08, C08F 12/24

(54) **Neat (solventless) hydrogenation of 4-acetoxyacetophenone in the production of 4-acetoxystyrene and its polymers and hydrolysis products.**

(30) Priority : **19.07.88 US 221145**

(43) Date of publication of application : **07.02.90 Bulletin 90/06**

(45) Publication of the grant of the patent : **30.12.92 Bulletin 92/53**

(84) Designated Contracting States : **AT BE CH DE ES FR GB GR IT LI LU NL SE**

(56) References cited :
**US-A- 2 748 160**
**THE JOURNAL OF ORGANIC CHEMISTRY, vol. 23, no. 4, 18th April 1958, pages 544-549, The American Chemical Society; B.B. CORSONet al.: "Preparation of vinylphenols and isopropenylphenols"**

(73) Proprietor : **HOECHST CELANESE CORPORATION**
**Route 202-206 North**
**Somerville, N.J. 08876 (US)**

(72) Inventor : **Shah, Bakulesh N.**
**3407 Wentletrap**
**Bay City Texas 77414 (US)**
Inventor : **Keene, Donna L.**
**Route 1 Box 215**
**Carrollton Virginia (US)**

(74) Representative : **De Minvielle-Devaux, Ian Benedict Peter et al**
**CARPMAELS & RANSFORD 43, Bloomsbury Square**
**London WC1A 2RA (GB)**

EP 0 353 898 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to a process for the neat hydrogenation of 4-acetoxyacetophenone in the production of 4-acetoxystyrene. The invention also pertains to a process for the polymerization, and hydrolysis thereof.

It is known in the art to produce monomers, homopolymers and copolymers of 4-acetoxystyrene and to hydrolyze the same to produce 4-hydroxystyrene derivatives or poly(4-hydroxystyrene). Such find use in the production of adhesives, coating compositions, photoresists and the like. The monomeric acetoxystyrene finds use as an intermediate in the production of such polymers as poly(4-acetoxystyrene) and poly(4-hydroxystyrene). These later compounds are useful as binder resins for photoresists. Alpha acetoxystyrene and beta acetoxystyrene are known per se and are described in U.S. patent 4,144,063 and acetoxymethylstyrene is taught in U.S. Patent 3,963,495. U.S. patent 4,075,237 describes 1,4-dimethyl 2-hydroxystyrene, while U.S. Patent 4,565,846 teaches the use of poly(3,5-dimethyl-4-hydroxystyrene).

Japanese patent 84023747 describes anti-static compounds employing poly-acetoxymethylstyrene and U.S. Patent 4,221,700 describes a stabilized synthetic polymer composition using poly(alkylated alkenylphenol) including 2-methyl paravinyl phenol. U.S. Patents 4,600,683 and 4,543,397 describe poly (alphamethyl vinylphenol). U.S. Patents 4,517,028; 4,460,770 and 4,539,051 describe dimethyl vinyl phenol. The preparation of 4-hydroxystyrene is well known in the art. One process is described in U.S. Patent 4,316,995 and another is described in U.S. Patent 4,451,676.

Vinyl phenol may be prepared by a five step process of (1) acetylating phenol to p-hydroxyacetophenone, (2) acetylation of p-hydroxyacetophenone to p-acetoxyacetophenone, (3) hydrogenation to p-acetoxyphenyl methylcarbinol, (4) dehydration to p-acetoxystyrene, and (5) saponification to p-vinylphenol. The method is more fully described in Corson, B. B., et al, Preparation of Vinylphenols and Isopropenylphenols, J. Org. Chem., April 1958.

The prior art process for the hydrogenation of 4-acetoxyacetophenone to acetoxyphenyl methylcarbinol has been conducted with a Pd/C catalyst. However, this method has required the presence of such solvents as methanol and tetrahydrofuran. The required use of the solvent has been a problem in practice of this process. Problems which have been experienced include the dissolution of the 4-acetoxyacetophenone in the solvent; increased reactor volume due to the presence of the solvent; subsequent removal of the Pd/C catalyst from the large volume of solvent in the 4-acetoxyphenyl methylcarbinol mixture; separation of the solvent from 4-acetoxyphenyl methylcarbinol; purification and recycling of the solvent; solvent losses; and by-products from the solvent. The present invention employs a process wherein the solvent is completely eliminated. It has been surprisingly found that the selectivity of the reaction is substantially not adversely affected by the elimination of the solvent component. Also, surprisingly other side reactions are not observed and an economical operation of the process is made possible.

### Summary of the Invention

The invention provides a method for producing 4-acetoxyphenyl methylcarbinol which comprises heating 4-acetoxyacetophenone at a temperature of from 54°C to 120°C, preferentially from 60°C to 90°C in the presence of at least a stoichiometric amount of hydrogen, and a catalyst selected from the group consisting of Pd/C or activated nickel such as Raney Nickel in the absence of a solvent, for a sufficient time to produce 4-acetoxyphenyl methylcarbinol. The invention also provides a process for the production of 4-acetoxystyrene which comprises:

a) acylating phenol with acetic anhydride to produce 4-hydroxyacetophenone; and

b) acylating the 4-hydroxyacetophenone with acetic anhydride to form 4-acetoxyacetophenone; and

c) heating 4-acetoxyacetophenone at a temperature of from 54°C to 120°C in the presence of at least a stoichiometric amount of hydrogen, and a catalyst selected from the group consisting of Pd/C or activated nickel such as Raney Nickel in the absence of a solvent, for a sufficient time to produce 4-acetoxyphenyl methylcarbinol; and

d) dehydrating the 4-acetoxyphenyl methylcarbinol to produce 4-acetoxystyrene.

The invention also provides a process for the production of poly(4-acetoxystyrene) which comprises subsequent free radical polymerization of the 4-acetoxystyrene to form poly(4-acetoxystyrene) having a molecular weight in the range of from about 1,000 to about 800,000, preferably about 5,000 to about 500,000.

The invention still further provides a process for the production of poly(4-hydroxystyrene) which comprises subsequently hydrolyzing the poly(4-acetoxystyrene) to form poly(4-hydroxystyrene) having a molecular weight in the range of from about 1,000 to about 500,000, preferably about 5,000 to about 500,000.

Detailed Description of the Preferred Embodiment

In the process for the production of 4-acetoxystyrene, one begins with phenol and acylates it with acetic anhydride and via a Friedel-Crafts catalysis or Fries rearrangement produces 4-hydroxyacetophenone. This 4-hydroxyacetophenone is then esterified with acetic anhydride to form 4-acetoxyacetophenone. The latter is then neat hydrogenated to form 4-acetoxyphenyl methylcarbinol. This is then dehydrated with an acid or base to form 4-acetoxystyrene monomer. Free radical polymerization and hydrolysis may follow.

A typical overall reaction sequence may be described schematically as follows:

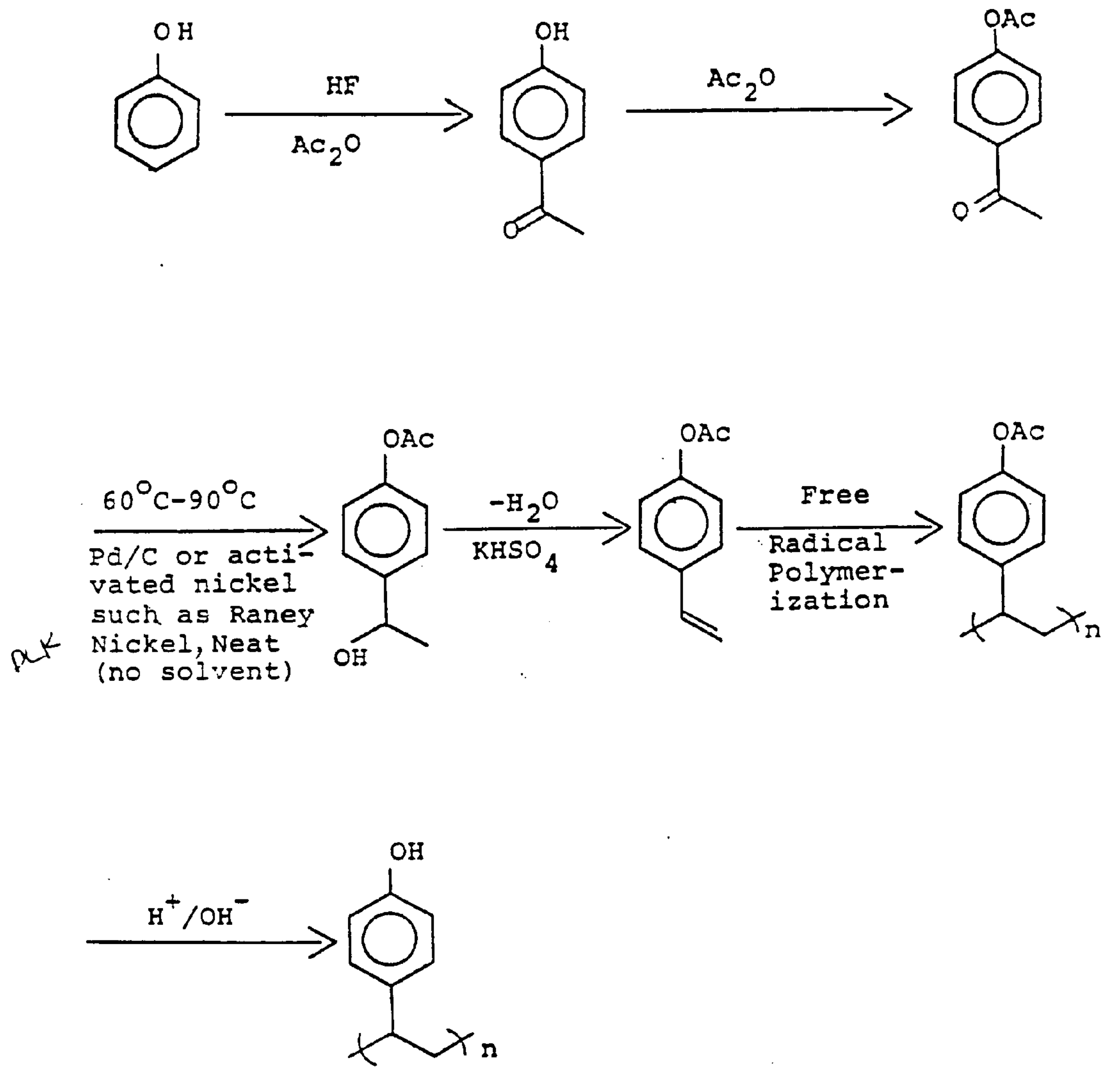


In the preferred embodiment the first reaction steps proceed as follows. One charges a reaction vessel with phenol and a slight excess of acetic anhydride and a Friedel-Crafts catalyst such as hydrogen fluoride. The acylation is conducted at a temperature of from about 5°C to about 100°C, or more preferably from about 20°C to about 80°C. A most preferred temperature is about 50°C. The reaction proceeds at a preferred pressure of from about 700 mm Hg to about 780 mm Hg for from about 1 to about 5 hours. Although hydrogen fluoride is the preferred catalyst, other Lewis acids may be also used such as $AlCl_3$, $BF_3$, $HClO_4$, $FeCl_3$ and $SnCl_4$. In the alternative, the acylation may be conducted by a Fries rearrangement, in a manner well known to the skilled artisan. The reaction product is 4-hydroxyacetophenone. This 4-hydroxyacetophenone is then esterified with acetic anhydride. In this process, the 4-hydroxyacetophenone is refluxed with

an excess of acetic anhydride for from about 2 to about 20 hours. Excess acetic anhydride as well as generated acetic acid are removed by distillation in vacuo. This is conducted, for example at a pressure of from about .1 to about 1000 mm HgA and at an initial temperature of from about 135°C to about 150°C, preferably from about 135°C to about 145°C which is then reduced to from about 35°C to about 85°C. The 4-acetoxyacetophenone is then neat hydrogenated to 4-acetoxyphenyl methylcarbinol. This is performed by heating 4-acetoxyacetophenone at a temperature of from 54°C to 120°C in the presence of at least a stoichiometric amount of hydrogen, and a catalyst selected from the group consisting of Pd/C or activated nickel such as Raney Nickel in the absence of a solvent, for a sufficient time to produce acetoxyphenyl methylcarbinol. In the more preferred embodiment, the reaction is conducted at a temperature of from 60°C to 90°C. In the preferred embodiment, the reaction is conducted until substantial completion of hydrogenation as indicated by a lack of $H_2$ uptake, normally about 2 to 7 hours. In the preferred embodiment, when Pd/C is used, the reaction proceeds at a pressure of from 14.7 psig to 5000 psig, more preferably at a pressure of from 50 psig to 500 psig and most preferably at a pressure of from 100 psig to 400 psig. When activated nickel is used, the reaction proceeds at a pressure of from 14.7 to 5000 psig, more preferably from 300 to 600 psig and most preferably from 350 to 450 psig. Activated nickel is preferred since it can be recycled and a process with a higher selectivity can be attained.

This product is then dehydrated. Dehydration is preferably conducted by vacuum heating in the presence of a polymerization inhibitor and a dehydrating agent. In one preferred embodiment, the 4-acetoxyphenyl methylcarbinol is mixed with a $KHSO_4$ dehydrating agent and t-butyl catechol as a polymerization inhibitor. Other useful dehydrating agents non-exclusively include bases, $CuSO_4$, $CuCl_2$ and $Al_2O_3$. Other polymerization inhibitors non-exclusively include hydroquinone, phenothiazine, tetrachloroquinone, tert-butyl catechol and di-t-butyl-p-cresol. The dehydrating agent is present in an amount of from about 0.25 to about 5.0 percent weight of the 4-acetoxyphenyl methylcarbinol. The polymerization inhibitor is preferably present in an amount of from about 1% to about 5% based on the weight of the methylcarbinol. The reaction vessel is heated to from about 160°C to about 230°C, preferably 170°C to about 190°C at a pressure of from about 0.1 to about 760 mm HgA. The resultant product is 4-acetoxystyrene with water as by product. The 4-acetoxystyrene monomer may then be polymerized by a free radical initiation process to produce poly(4-acetoxystyrene) such that it has a molecular weight in the range of from about 1,000 to about 800,000, preferably 5,000 to 500,000 or more preferably about 5,000 to about 300,000. This intermediate is then hydrolyzed with a base or acid to form poly(4-hydroxystyrene) such that it also has the aforesaid molecular weight range. One preferred free radical initiator is azobisisobutyronitrile. Other azo type initiators are also suitable. Still others non-exclusively include peroxides such as benzoyl peroxide, and di-t-butyl peroxide. It is predicted that essentially any free radical initiation system will serve in the same fashion. One preferred hydrolyzing agent is tetramethyl ammonium hydroxide. Other hydrolyzing agents non-exclusively include aqueous $NH_3$, NaOH, KOH, HCl, and $H_2SO_4$.

The following non-limiting examples serve to illustrate the invention.

## Example 1

75 grams of 4-acetoxyacetophenone and 7.5g of Pd/C catalyst (5% Pd carbon) are charged in a 300-ml stirred reactor. The reactor is pressure checked with nitrogen at 150 psig. The nitrogen is then purged twice with 100 psig hydrogen. The hydrogen pressure is maintained at 100 psig. The reactor is then heated to 60°C to carry out the reaction. After three hours of reaction time, the reaction is stopped by venting off hydrogen. A sample of the reaction mass is analyzed by gas chromatography. The analysis shows 4-acetoxyphenyl methylcarbinol 77.4%, unreacted 4-acetoxyacetophenone 6.6%, 4-ethylphenol 1.5%, 4-ethylphenyl acetate 0.2% and the balance is as due to other components.

## Example 2

12 kg of 4-acetoxyacetophenone and 450g of Pd/C catalyst (5% Pd on carbon) are charged in a 5-gal stirred reactor. The 4-acetoxyacetophenone is melted before charging in the reactor. After pressure testing with nitrogen, the reactor is charged with hydrogen at 100 psig and the reactor contents are heated to 60°C. After 8 hours, the heat is turned off to stop the reaction. The product analysis by gas chromatography gives 92.3% 4-acetoxyphenyl methylcarbinol, 1.8% 4-ethylphenyl acetate, 0.3% 4-acetoxyacetophenone, 1.6% ethylbenzene and the balance is due to other components.

## Example 3

12 kg of 4-acetoxyacetophenone and 450g of Pd/C catalyst (5% Pd on carbon) are charged in a 5-gal stirred reactor. The reactor is then charged with hydrogen at 80 psig and the reactor contents are then heated to 60°C.

After 8 hours of reaction time, the reaction is stopped. The product analysis by gas chromatography gives 94% 4-acetoxyphenyl methylcarbinol, 1.3% ethylphenol, 1.2% 4-ethylphenyl acetate, 2.5% 4-acetoxyacetophenone and the balance is due to other components.

Example 4

102g of 4-acetoxyacetophenone and 10g of Raney nickel catalyst are charged in a 300-ml stirred reactor. The reactor is then charged with hydrogen at 500 psig and heated to 60°C for 270 minutes. The reactor is then cooled down and the product analyzed by gas chromatography. The analysis gives 80.4% 4-acetoxyphenyl methylcarbinol.

Example 5

99.8g of 4-acetoxyacetophenone and 4g of Pd/C catalyst (5% Pd on carbon) are charged in a 300-ml stirred reactor. The reactor is then charged with hydrogen at 100 psig. The contents are heated to 60°C. After 4.25 hours, the reactor is cooled down and the product is analyzed by gas chromatography. The analysis is 94% acetoxyphenyl methylcarbinol, 2% ethylbenzene, 0.7% 4-ethylphenyl acetate and the balance is due to other components.

Example 6

10 kg of melted 4-acetoxyacetophenone and 1.1 kg of Raney nickel catalyst are charged in a 20-l stirred reactor. The contents are heated to 60°C at 400 psig of nitrogen. The reactor is then charged with hydrogen. After 4 hours, the reactor is cooled down and the liquid product is analyzed by gas chromatography. The analysis is 97% acetoxyphenyl methylcarbinol, 0.1% ethylbenzene, 0.2% ethylphenyl acetate, 1.3% 4-acetoxyacetophenone and the balance is due to other components.

## Claims

1. A method for producing 4-acetoxyphenyl methylcarbinol which comprises heating 4-acetoxyacetophenone at a temperature of from 54°C to 120°C in the presence of at least a stoichiometric amount of hydrogen, and a catalyst selected from the group consisting of Pd/C or activated nickel in the absence of a solvent, for a sufficient time to produce 4-acetoxyphenyl methylcarbinol.

2. The method of claim 1 wherein the catalyst is Pd/C.

3. The method of claim 1 wherein the catalyst is Raney Nickel.

4. The method of claim 1 wherein the reaction is conducted until substantial completion of hydrogenation.

5. The method of claim 1 wherein the reaction is conducted at a pressure of from 14 psig to 5000 psig.

6. A process for the production of 4-acetoxystyrene which comprises:
   a) acylating phenol with acetic anhydride to produce 4-hydroxyacetophenone; and
   b) acylating the 4-hydroxyacetophenone with acetic anhydride to form 4-acetoxyacetophenone; and
   c) heating 4-acetoxyacetophenone at a temperature of from 54°C to 120°C in the presence of at least a stoichiometric amount of hydrogen, and a catalyst selected from the group consisting of Pd/C or activated nickel in the absence of a solvent, for a sufficient time to produce 4-acetoxyphenyl methylcarbinol; and
   d) dehydrating the 4-acetoxyphenyl methylcarbinol to produce 4-acetoxystyrene.

7. The method of claim 6 wherein the catalyst is Pd/C.

8. The method of claim 6 wherein the catalyst is Raney Nickel.

9. The method of claim 6 wherein the reaction step (c) is conducted until substantial completion of hydrogenation.

10. The method of claim 6 wherein the reaction step (c) is conducted at a pressure of from 14 psig to 5,000 psig.

11. A process for the production of poly(4-acetoxystyrene) which comprises:
a) acylating phenol with acetic anhydride to produce 4-hydroxyacetophenone; and
b) acylating the 4-hydroxyacetophenone with acetic anhydride to form 4-acetoxyacetophenone; and
c) heating 4-acetoxyacetophenone at a temperature of from 54°C to 120°C in the presence of at least a stoichiometric amount of hydrogen, and a catalytic amount of a catalyst selected from the group consisting of Pd/C or activated nickel in the absence of a solvent, for a sufficient time to produce 4-acetoxyphenyl methylcarbinol; and
d) dehydrating the 4-acetoxyphenyl methylcarbinol to produce 4-acetoxystyrene, and
e) free radical polymerizing the 4-acetoxystyrene to form poly(4-acetoxystyrene) having a molecular weight in the range of from 1,000 to 800,000.

12. The method of claim 11 wherein the catalyst is Pd/C.

13. The method of claim 11 wherein the catalyst is Raney Nickel.

14. The method of claim 11 wherein the reaction step (c) is conducted until substantial completion of hydrogenation.

15. The method of claim 11 wherein the reaction step (c) is conducted at a pressure of from 14 psig to 5,000 psig.

16. A process for the production of poly(4-hydroxystyrene) which comprises:
a) acylating phenol with acetic anhydride to produce 4-hydroxyacetophenone; and
b) acylating the 4-hydroxyacetophenone with acetic anhydride to form 4-acetoxyacetophenone; and
c) heating 4-acetoxyacetophenone at a temperature of from 54°C to 120°C in the presence of at least a stoichiometric amount of hydrogen, and a catalytic amount of a catalyst selected from the group consisting of Pd/C or activated nickel in the absence of a solvent, for a sufficient time to produce 4-acetoxyphenyl methylcarbinol; and
d) dehydrating the 4-acetoxyphenyl methylcarbinol to produce 4-acetoxystyrene, and
e) free radical polymerizing the 4-acetoxystyrene to form poly(4-acetoxystyrene) having a molecular weight in the range of from 1,000 to 800,000; and
f) hydrolyzing the poly(4-acetoxystyrene) to form poly(4-hydroxystyrene) having a molecular weight in the range of from 1,000 to 500,000.

17. The method of claim 16 wherein the catalyst is Pd/C.

18. The method of claim 16 wherein the catalyst is Raney Nickel.

19. The method of claim 16 wherein the reaction step (c) is conducted until substantial completion of hydrogenation.

20. The method of claim 16 wherein the reaction step (c) is conducted at a pressure of from 14 psig to 5,000 psig.

**Patentansprüche**

1. Verfahren zur Herstellung von 4-Acetoxyphenylmethylcarbinol umfassend das Erwärmen von 4-Acetoxyacetophenon auf eine Temperatur von 54 °C bis 120 °C in Gegenwart von wenigstens einer stöchiometrischen Wasserstoffmenge und einem Katalysator, ausgewählt aus der Gruppe von Pd/C oder aktiviertem Nickel in Abwesenheit eines Lösungsmittels, für eine Zeit, die ausreicht, 4-Acetoxyphenylmethylcarbinol zu produzieren.

2. Verfahren nach Anspruch 1, worin der Katalysator Pd/C ist.

3. Verfahren nach Anspruch 1, worin der Katalysator Raney-Nickel ist.

4. Verfahren nach Anspruch 1, worin die Reaktion bis zur im wesentlichen Vervollständigung der Hydrierung durchgeführt wird.

5. Verfahren nach Anspruch 1, worin die Reaktion bei einem Druck von 14 psig bis 5000 psig durchgeführt wird.

6. Verfahren zur Herstellung von 4-Acetoxystyrol umfassend:
a) Acylierung von Phenol mit Essigsäureanhydrid zur Herstellung von 4-Hydroxyacetophenon; und
b) Acylierung von 4-Hydroxyacetophenon mit Essigsäureanhydrid zur Bildung von 4-Acetoxyacetophenon; und
c) Erwärmung von 4-Acetoxyacetophenon bei einer Temperatur von 54 °C bis 120 °C in Gegenwart von wenigstens einer stöchiometrischen Wasserstoffmenge und einem Katalysator, ausgewählt aus der Gruppe von Pd/C oder aktiviertem Nickel, in Abwesenheit eines Lösungsmittels, für eine Zeit, die ausreicht, 4-Acetoxyphenylmethylcarbinol zu produzieren und
d) Dehydratisierung von 4-Acetoxyphenylmethylcarbinol zur Gewinnung von 4-Acetoxystyrol.

7. Verfahren nach Anspruch 6, worin der Katalysator Pd/C ist.

8. Verfahren nach Anspruch 6, worin der Katalysator Raney-Nickel ist.

9. Verfahren nach Anspruch 6, worin die Reaktionsstufe (c) bis zur im wesentlichen Vervollständigung der Hydrierung durchgeführt wird.

10. Verfahren nach Anspruch 6, worin die Reaktionsstufe (c) bei einem Druck von 14 psig bis 5000 psig durchgeführt wird.

11. Verfahren zur Herstellung von Poly(4-acetoxystyrol) umfassend:
a) Acylierung von Phenol mit Essigsäureanhydrid zur Bildung von 4-Hydroxyacetophenon; und
b) Acylierung von 4-Hydroxyacetophenon mit Essigsäureanhydrid zur Bildung von 4-Acetoxyacetophenon; und
c) Erwärmung von 4-Acetoxyacetophenon bei einer Temperatur von 54 °C bis 120 °C in Gegenwart von wenigstens einer stöchiometrischen Wasserstoffmenge und einer katalytischen Menge eines Katalysators, ausgewählt aus der Gruppe von Pd/C oder aktiviertem Nickel, in Abwesenheit eines Lösungsmittels, für eine Zeit, die ausreicht, 4-Acetoxyphenylmethylcarbinol zu produzieren und
d) Dehydratisierung von 4-Acetoxyphenylmethylcarbinol zur Gewinnung von 4-Acetoxystyrol und
e) freie Radikalpolymerisation von 4-Acetostyrol zur Bildung von Poly(4-Acetostyrol), das ein Molekulargewicht im Bereich von 1 000 bis 800 000 aufweist.

12. Verfahren nach Anspruch 11, worin der Katalysator Pd/C ist.

13. Verfahren nach Anspruch 11, worin der Katalysator Raney-Nickel ist.

14. Verfahren nach Anspruch 11, worin die Reaktionsstufe (c) bis zur im wesentlichen Vervollständigung der Hydrierung durchgeführt wird.

15. Verfahren nach Anspruch 11, worin die Reaktionsstufe (c) bei einem Druck von 14 psig bis 5000 psig durchgeführt wird.

16. Verfahren zur Herstellung von Poly(4-Hydroxystyrol) umfassend:
a) Acylierung von Phenol mit Essigsäureanhydrid zur Bildung von 4-Hydroxyacetophenon; und
b) Acylierung von 4-Hydroxyacetophenon mit Essigsäureanhydrid zur Bildung von 4-Acetoxyacetophenon; und
c) Erwärmung von 4-Acetoxyacetophenon bei einer Temperatur von 54 °C bis 120 °C in Gegenwart von wenigstens einer stöchiometrischen Wasserstoffmenge und einer katalytischen Menge eines Katalysators, ausgewählt aus der Gruppe von Pd/C oder aktiviertem Nickel, in Abwesenheit eines Lösungsmittels, für eine Zeit, die ausreicht, 4-Acetoxyphenylmethylcarbinol zu produzieren und
d) Dehydratisierung von 4-Acetoxyphenylmethylcarbinol zur Gewinnung von 4-Acetoxystyrol und
e) freie Radikalpolymerisation von 4-Acetostyrol zur Bildung von Poly(4-Acetostyrol), das ein Molekulargewicht im Bereich von 1 000 bis 800 000 aufweist und
f) Hydrolyse von Poly(4-acetoxystyrol) zur Bildung von Poly(4-hydroxystyrol), das ein Molekulargewicht

von 1 000 bis 500 000 aufweist.

**17.** Verfahren nach Anspruch 16, worin der Katalysator Pd/C ist.

**18.** Verfahren nach Anspruch 16, worin der Katalysator Raney-Nickel ist.

**19.** Verfahren nach Anspruch 16, worin die Reaktionsstufe (c) bis zur im wesentlichen Vervollständigung der Hydrierung durchgeführt wird.

**20.** Verfahren nach Anspruch 16, worin die Reaktionsstufe (c) bei einem Druck von 14 psig bis 5000 psig durchgeführt wird.

## Revendications

**1.** Procédé de production de 4-acétoxyphénylméthylcarbinol, qui consiste à chauffer de la 4-acétoxyacétophénone à une température de 54°C à 120°C en présence d'au moins une quantité stoechiométrique d'hydrogène et d'un catalyseur choisi dans le groupe comprenant Pd/C ou du nickel activé en l'absence d'un solvant, pendant une durée suffisante pour produire du 4-acétoxyphénylméthylcarbinol.

**2.** Procédé suivant la revendication 1, dans lequel le catalyseur est Pd/C.

**3.** Procédé suivant la revendication 1, dans lequel le catalyseur est le nickel de Raney.

**4.** Procédé suivant la revendication 1, dans lequel la réaction est conduite jusqu'à ce que l'hydrogénation soit principalement achevée.

**5.** Procédé suivant la revendication 1, dans lequel la réaction est conduite à une pression manométrique allant de 14 à 5000 $lb/in^2$.

**6.** Procédé de production de 4-acétoxystyrène, qui consiste :
a) à acyler du phénol avec de l'anhydride acétique pour produire de la 4-hydroxyacétophénone ; et
b) à acyler la 4-hydroxyacétophénone avec de l'anhydride acétique pour former la 4-acétoxyacétophénone ; et
c) à chauffer la 4-acétoxyacétophénone à une température de 54 à 120°C en présence d'au moins une quantité stoechiométrique d'hydrogène et d'un catalyseur choisi dans le groupe comprenant Pd/C ou le nickel activé, en l'absence d'un solvant, pendant une durée suffisante pour produire le 4-acétoxyphénylméthylcarbinol ; et
d) à déshydrater le 4-acétoxyphénylméthylcarbinol pour produire le 4-acétoxystyrène.

**7.** Procédé suivant la revendication 6, dans lequel le catalyseur est Pd/C.

**8.** Procédé suivant la revendication 6, dans lequel le catalyseur est le nickel de Raney.

**9.** Procédé suivant la revendication 6, dans lequel l'étape réactionnelle (c) est conduite jusqu'à ce que l'hydrogénation soit principalement achevée.

**10.** Procédé suivant la revendication 6, dans lequel l'étape réactionnelle (c) est conduite à une pression manométrique d'environ 14 à 5000 $lb/in^2$.

**11.** Procédé de production d'un poly(4-acétoxystyrène), qui consiste :
a) à acyler du phénol avec de l'anhydride acétique pour produire de la 4-hydroxyacétophénone ; et
b) à acyler la 4-hydroxyacétophénone avec l'anhydride acétique pour former la 4-acétoxyacétophénone ; et
c) à chauffer la 4-acétoxyacétophénone à une température de 54 à 120°C en présence d'une quantité au moins stoechiométrique d'hydrogène et d'une quantité catalytique d'un catalyseur choisi dans le groupe comprenant Pd/C ou du nickel activé en l'absence d'un solvant, pendant une durée suffisante pour produire du 4-acétoxyphénylméthylcarbinol ; et
d) à déshydrater le 4-acétoxyphénylméthylcarbinol pour produire du 4-acétoxystyrène, et
e) à polymériser par radicaux libres le 4-acétoxystyrène pour former un poly(4-acétoxystyrène) ayant

un poids moléculaire compris dans la plage de 1000 à 800 000.

**12.** Procédé suivant la revendication 11, dans lequel le catalyseur est Pd/C.

**13.** Procédé suivant la revendication 11, dans lequel le catalyseur est le nickel de Raney.

**14.** Procédé suivant la revendication 11, dans lequel l'étape réactionnelle (c) est conduite jusqu'à ce que l'hydrogénation soit principalement achevée.

**15.** Procédé suivant la revendication 11, dans lequel l'étape réactionnelle (c) est conduite à une pression manométrique de 14 à 5000 lb/in$^2$.

**16.** Procédé de production d'un poly(4-hydroxystyrène), qui consiste :

a) à acyler du phénol avec de l'anhydride acétique pour produire la 4-hydroxyacétophénone ; et

b) à acyler la 4-hydroxyacétophénone avec de l'anhydride acétique pour former la 4-acétoxyacétophénone ; et

c) à chauffer la 4-acétoxyacétophénone à une température de 54 à 120°C en présence d'une quantité au moins stoéchiométrique d'hydrogène et d'une quantité catalytique d'un catalyseur choisi dans le groupe comprenant Pd/C ou du nickel activé en l'absence d'un solvant, pendant une durée suffisante pour produire du 4-acétoxyphénylméthylcarbinol ; et

d) à déshydrater le 4-acétoxyphénylméthylcarbinol pour produire du 4-acétoxystyrène, et

e) à polymériser par radicaux libres le 4-acétoxystyrène pour former un poly(4-acétoxystyrène) ayant un poids moléculaire compris dans la plage de 1000 à 800 000 ; et

f) à hydrolyser le poly(4-acétoxystyrène) pour former un poly(4-hydroxystyrène) ayant un poids moléculaire compris dans la plage de 1000 à 500 000.

**17.** Procédé suivant la revendication 16, dans lequel le catalyseur est Pd/C.

**18.** Procédé suivant la revendication 16, dans lequel le catalyseur est le nickel de Raney.

**19.** Procédé suivant la revendication 16, dans lequel l'étape réactionnelle (c) est conduite jusqu'à ce que l'hydrogénation soit principalement achevée.

**20.** Procédé suivant la revendication 16, dans lequel l'étape réactionnelle (c) est conduite à une pression manométrique de 14 à 5000 lb/in$^2$.